(19) Europäisches Patentamt
European Patent Office
Office européen des brevets

(11) Veröffentlichungsnummer: **0 011 207**
**B1**

(12) # EUROPÄISCHE PATENTSCHRIFT

(45) Veröffentlichungstag der Patentschrift: **25.11.81**

(51) Int. Cl.³: **C 07 C 59/185**, C 07 C 51/373

(21) Anmeldenummer: **79104307.8**

(22) Anmeldetag **05.11.79**

(54) **Verfahren zur Herstellung von 3,3-Dimethyl-2-oxobuttersäure.**

(30) Priorität: **13.11.78 US 960330**

(43) Veröffentlichungstag der Anmeldung: **28.05.80 Patentblatt 80/11**

(45) Bekanntmachung des Hinweises auf die Patenterteilung **25.11.81 Patentblatt 81/47**

(84) Benannte Vertragsstaaten: **BE CH DE FR GB IT NL**

(56) Entgegenhaltungen:
**DE-A1-2 642 671**
**DE-A1-2 648 300**
**Chemical Abstracts Band 74, Nr. 8, 1971 Columbus, Ohio, USA S. WOLFE et al. »Ruthenium trichloride-catalyzed hypochlorite oxidation of organic compounds« Seite 245, Spalte 2, Abstract Nr. 35102q**

(73) Patentinhaber: **Mobay Chemical Corporation, Penn Lincoln Parkway West, Pittsburgh, Pennsylvania 15205 (US)**

(72) Erfinder: **Jackman, Dennis Earl, 4705 W. 71st Terrace, Prairie Village, Kansas 66208 (US)**

(74) Vertreter: **Gremm, Joachim, Dr. et al, c/o Bayer AG, Zentralbereich Patente Marken und Lizenzen, D-5090 Leverkusen, Bayerwerk (DE)**

**EP 0 011 207 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von 3,3-Dimethyl-2-oxobuttersäure

Die Erfindung betrifft ein Verfahren zur Herstellung von 3,3-Dimethyl-2-oxobuttersäure und ihren Salzen durch katalytische Oxidation von 3,3-Dimethyl-2-hydroxybuttersäure.

3,3-Dimethyl-2-oxobuttersäure wird benötigt als Zwischenprodukt zur großtechnischen Herstellung — durch Umsetzung mit Thiocarbohydrazid und anschließende S-Methylierung — von 4-Amino-6-tert.-butyl-3-methylthio-1,2,4-triazin-5(4H)-on (Metribuzin), einem selektiven Herbizid, das vornehmlich in Kulturen von Sojabohnen, Tomaten und Kartoffeln eingesetzt wird (vgl. Chem. Ber. 97, 2173—8 [1964]; US-PS 3 671 523 und US-PS 3 905 801). 3,3-Dimethyl-2-oxobuttersäure kann hergestellt werden durch Oxidation von 3,3-Dimethyl-2-hydroxybuttersäure mit Kaliumpermanganat (vgl. DE-OS 2 648 300).

Dieses Verfahren ist zwar auch für großtechnische Durchführung durchaus geeignet und ist bisher zur Herstellung von großen Mengen Metribuzin verwendet worden, es ist jedoch wegen des hohen Preises für Kaliumpermanganat sehr teuer; außerdem fallen große Mengen an Mangandioxid (Braunstein) als Nebenprodukt an, die deponiert werden müssen und gegebenenfalls zur Umweltbelastung beitragen können.

Ziel der Erfindung ist daher die Schaffung eines verbesserten und wirtschaftlicheren Verfahrens zur Herstellung von 3,3-Dimethyl-2-oxobuttersäure und/oder ihren Salzen.

Dieses Ziel und andere Vorteile werden erfindungsgemäß dadurch erreicht, daß man 3,3-Dimethyl-2-hydroxybuttersäure

$$(CH_3)_3C-\underset{\displaystyle OH}{\underset{|}{CH}}-COOH$$

(»Hydroxysäure«)

in wäßrig-alkalischer Lösung mit der zumindest stöchiometrischen Menge eines Salzes der unterchlorigen Säure (Hypochlorit) in Gegenwart eines Rutheniumkatalysators oxidiert und die 3,3-Dimethyl-2-oxobuttersäure

$$(CH_3)_3C-\underset{\displaystyle O}{\underset{||}{C}}-COOH$$

(»Ketosäure«)

gegebenenfalls aus der zunächst erhaltenen Lösung ihrer Salze in Freiheit setzt.

Günstigerweise werden zu diesem Zweck die Natriumsalze der genannten Säuren eingesetzt, es können jedoch auch andere Alkali- und Erdalkalimetallsalze eingesetzt werden, mit der Maßgabe, daß sie im wäßrigen Reaktionsmedium löslich sind.

Das Reaktionsmedium weist vorteilhaft einen pH-Wert von etwa 9 – 13, vorzugsweise von etwa 10—12 auf. Da ein Teil der Hydroxylionen im Verlauf der Umsetzung verbraucht zu werden scheint, wird Alkali entweder im Überschuß eingesetzt oder während der Oxidation zugegeben, um den gewünschten pH-Wert aufrecht zu erhalten. Wenn der pH-Wert der Lösung des Salzes der 3,3-Dimethyl-2-hydroxybuttersäure (»Hydroxysäure«) unter 6,0 abfällt oder der Gehalt der Hypochloritlösung an freiem Ätzalkali auf weniger als etwa 1,3% absinkt, läuft die gewünschte Oxidation nicht in dem erforderlichen Ausmaß ab und es führt eine weitere Zugabe von Hypochlorit nur zur Spaltung von gegebenenfalls vorhandener Ketosäure. Außerdem wird der Ru-Katalysator in eine wasserlösliche Form umgewandelt, die aus der Lösung nur schwer abzutrennen ist.

Das Hypochlorit wird vorzugsweise in einem Überschuß von etwa 5—15% eingesetzt, um die vollständige Oxidation der 2-Hydroxysäure zur 2-Ketosäure zu gewährleisten. Das Hypochlorit kann in situ, z. B. durch Einleiten von Chlorgas in eine wäßrige, alkalische Lösung von »Hydroxysäure«, die den Katalysator enthält, hergestellt werden.

Die Umsetzung kann bei Raumtemperatur vorgenommen werden, wird jedoch zur Beschleunigung günstiger bei erhöhter Temperatur, vorzugsweise im Bereich von etwa 40 bis 60°C, durchgeführt. Temperaturen oberhalb von 60°C sind ungünstig und sollten vermieden werden, da in diesem Bereich infolge Decarbonylierung Pivalinsäure als Nebenprodukt gebildet wird.

Der Rutheniumkatalysator wird vom Hypochlorit zu einem Gemisch aus Ruthenat, Perruthenat und Rutheniumtetroxid oxidiert, wobei das Ruthenat überwiegt. Am Ende der Umsetzung liegt das Rutheniumoxid in Form eines Feststoffes in einer der ursprünglich eingesetzten im wesentlichen entsprechenden Menge vor, so daß es abfiltriert und selbst ohne Behandlung in einem weiteren Oxidations-Ansatz eingesetzt werden kann. Das Ruthenium kann in die Reaktionslösung in Form eines Salzes oder Oxides eingebracht werden, wobei Rutheniumdioxid und insbesondere Rutheniumdioxidhydrat bevorzugt wird. Das Oxid könnte z. B. in situ ausgehend von einem Salz, wie z. B. Rutheniumtrichlorid, hergestellt werden.

Das Rutheniumoxid wird in katalytischen Mengen, z. B. 0,01 bis etwa 1,0 g/Mol, vorzugsweise etwa 0,1—0,5 g/Mol »Hydroxysäure«, eingesetzt.

Die den Katalysator enthaltende, wäßrige »Hydroxysäure«-Salz-Lösung wird auf den gewünschten pH-Wert und die gewünschte Temperatur gebracht. Dann werden wäßrige Natronlauge zugegeben und Chlor eingeleitet oder zuvor hergestelltes Natriumhypochlorit, NaOCl, in Form einer wäßrigen Lösung zugetropft oder in Teilmengen zugegeben. Nach beendeter

Zugabe des gesamten Oxidationsmittels wird das Reaktionsgemisch bis zum Ende der Umsetzung stehen gelassen und dann abfiltriert, um den Katalysator aus der Lösung abzutrennen, die das gewünschte Produkt, d. h. ein Salz der 3,3-Dimethyl-2-oxobuttersäure, in hoher Ausbeute und Reinheit enthält.

Aus dieser Lösung kann die »Ketosäure« in üblicher Weise durch Ansäuern mit einer Mineralsäure, z. B. Salzsäure, in Freiheit gesetzt und ebenfalls in hoher Ausbeute und Reinheit isoliert werden.

Die Oxidation geht rasch vor sich und eine Zugabe des Hypochlorits innerhalb von nur 5 Minuten führt bereits zu sehr hohen Ausbeuten. Vorteilhaft werden jedoch längere Reaktionszeiten von 30 Minuten bis zu 1 Stunde eingehalten, um zu ermöglichen, daß der Rutheniumkatalysator in eine geeignete unlösliche Form überführt wird, so daß er abfiltriert und neuerlich in einem weiteren Reaktionsansatz verwendet werden kann. Die durch einfache Filtration erhaltene Lösung kann unmittelbar für die Umsetzung mit Thiocarbohydrazid zur Bildung von 4-Amino-6-tert.-butyl-3-thio-1,2,4-triazin-5(4H)-on eingesetzt werden.

In »Chemical Communications«, 1420 (1970) ist beschrieben, daß Verbindungen, welche die Gruppierung $-CHOH-CO-$ aufweisen, im Verlauf der Oxidation unter Einsatz von Ruthenium als Katalysator einer Kohlenstoff-Kohlenstoff-Spaltung und nicht der Überführung in $-CO-CO-$ unterliegen, z. B.:

$$-CHOH-CO- \longrightarrow -CHO + OHC-$$

$$-CHOH-CO- \longrightarrow -CO-CO-$$

Überraschend geht jedoch unter Einsatz der erfindungsgemäß vorgesehenen Ausgangsverbindungen in alkalisch-wäßrigem Reaktionsmedium die Oxidation unter Überführung der 2-Hydroxygruppe in eine 2-Carbonylgruppe ohne Spaltvorgang zwischen den Hydroxy bzw. Carbonyl tragenden Kohlenstoff-Atomen C-1 und C-2 vor sich.

Die Erfindung wird nachstehend anhand von Ausführungsbeispielen näher erläutert.

### Beispiel 1

a) In einen Rundkolben mit einem Fassungsraum von 2 Liter, der mit einem mechanischen Rührwerk, Thermometer, Kondensator und Tropftrichter versehen war, wurden 559 g einer 11,8%igen, wäßrigen Lösung von 3,3-Dimethyl-2-hydroxybuttersäure (»Hydroxysäure«) in Form des Natriumsalzes (0,5 Mol) und 0,2 g Rutheniumdioxidhydrat ($RuO_2 \cdot H_2O$) eingebracht. Der pH-Wert wurde auf 12 und die Temperatur auf 40°C eingestellt. Dann wurden unter raschem Rühren 330,5 g 12,1%iges NaOCl (0,5 Mol + 7,5% Überschuß) in Wasser innerhalb von 30 Minuten zugetropft, die Temperatur wurde während dieses Zeitraums mittels Eisbad auf 40°C gehalten. Nach beendeter Zugabe wurde das Eisbad entfernt, die Lösung 1 Stunde lang gerührt und dann filtriert, um den Katalysator zu entfernen. Es wurden 879 g einer 7,5%igen wäßrigen Lösung der 3,3-Dimethyl-2-oxobuttersäure (»Ketosäure«) als Natriumsalz erhalten. Ausbeute: etwa 100%.

b) Nach beendeter Oxidation lag der Ru-Katalysator in Form des schwarzen, wasserunlöslichen Rutheniumdioxidhydrats vor. Er wurde unter Einsatz von Celite-Filtrationshilfsmittel abfiltriert. Der nasse, aus Katalyator und Filtrationshilfsmittel bestehende Filterkuchen wurde unmittelbar in eine weitere Teilmenge von Hydroxysäuresalzlösung eingebracht und wie vorstehend beschrieben, wurde unter Zugabe von Hypochlorit eine weitere Oxidation durchgeführt.

c) 956 g einer 15,8%igen Thiocarbohydrazidlösung in verdünntem HCl wurden innerhalb von 10 Minuten unter raschem Rühren bei 70°C mit 2682 g 7,27%igem »Ketosäure«-filtrat (erwärmt auf 70°C) versetzt, das wie unter a) beschrieben hergestellt worden war. Nach vierstündigem Halten der Temperatur auf 70°C bei einem pH-Wert von 1,3 wurde die Lösung abgekühlt und abfiltriert. Der erhaltene Feststoff wurde mit Wasser gewaschen und luftgetrocknet. Es wurden 272,2 g (99,3% reines) 4-Amino-6-tert.-butyl-3-thio-1,2,4-triazin-5(4H)-on erhalten.

### Beispiel 2

In einen mit Rührwerk, Thermometer und Zufuhrtrichter versehenen Vierhals-Rundkolben mit einem Fassungsraum von 1 Liter wurden 524 g einer wäßrigen Lösung mit einem Gehalt von 0,5 Mol »Hydroxysäure« und 100 mg Rutheniumdioxidhydrat eingebracht. Die Temperatur wurde mittels Eisbad auf 15°C gehalten, es wurden unter Rühren innerhalb von 1,5 Stunden 294 g 11,9%ige NaOCl-Lösung zugetropft. Danach wurde die Lösung innerhalb von 1 Stunde unter Rühren auf Raumtemperatur gebracht. Nach Filtration durch Glasfaserfilterpapier (Handelsbezeichnung GFA) und Waschen des $RuO_2$ mit einer kleinen Menge verdünnter Natronlauge wurden 829 g einer Lösung mit einem Gehalt von 7,25% »Ketosäure« erhalten. Trotz der niedrigen Reaktionstemperatur und der geringen Katalysatormenge betrug die Ausbeute 94,4%.

### Beispiel 3
### (Vergleichsbeispiel)

Es wurde wie in Beispiel 1 beschrieben vorgegangen, jedoch bei einer Temperatur von 80°C gearbeitet. 427,4 g (3,5% Überschuß) 9,0%iges NaOCl wurden innerhalb von etwa

30 Minuten zu 555 g 11,7%iger »Hydroxysäure« mit einem Gehalt von 0,4 g $RuO_2 \cdot H_2O$ zugegeben. Nach Filtration wurden 982 g einer Lösung mit einem Gehalt von 5,82% »Ketosäure« (87,9%), 0,44% nicht umgesetzte »Hydroxysäure« (6,5%) und 0,32% (6,6% Ausbeute) Pivalinsäure in Form ihres Salzes erhalten. — Daraus geht hervor, daß bei höheren Temperaturen kleine Mengen Pivalinsäure als Nebenprodukt anfallen.

Beispiel 4

Es wurde wie in Beispiel 1 vorgegangen; das NaOCl wurde mit konstanter Geschwindigkeit innerhalb von 5 Minuten zugegeben, während die Temperatur des Reaktionsgemisches auf 40°C gehalten wurde. Es wurden 982 g einer Lösung mit einem Gehalt von 6,36% »Ketosäure« (96,1% Ausbeute) erhalten, die keine nicht umgesetzte »Hydroxysäure« mehr enthielt. Es wurden 0,3 g $RuO_2 \cdot H_2O$ eingesetzt. Infolge der niedrigen Temperatur wurde die Bildung von Pivalinsäure vermieden, die im Vergleich zu Beispiel 1 schnellere Zugabe führte jedoch zu einem geringen Ausbeuteverlust.

Beispiel 5

Beispiel 4 wurde wiederholt, jedoch wurde die NaOCl-Lösung innerhalb von 2 Stunden zugegeben und es wurden 983 g einer Lösung mit einem Gehalt von 6,55% »Ketosäure« (Ausbeute 99%) erhalten.

Beispiel 6

Beispiel 1 wurde unter Einsatz von an ein pH-Meßgerät (Modell Sargent-Welch LS) angeschlossener Glas- und Platinelektrode und eines 10 mV-Schreibers wiederholt. Das NaOCl wurde bei konstanter Geschwindigkeit mit einer Meßpumpe zugeführt. Das Potential der Lösung wurde auf 330—400 mV gehalten. Nahe dem Ende der Umsetzung stieg die Spannung auf 500 mV an, an diesem Punkt wurde die NaOCl-Zugabe eingestellt. Es wurden aus 559 g 11,8%iger »Hydroxysäurelösung« 921 g einer Lösung mit einem Gehalt von 6,7% »Ketosäure« (Ausbeute 95%) erhalten.

Beispiel 7

Zu einem Gemisch aus 120 ml Wasser, 100 ml 50%iger Natriumhydroxidlösung, 1 g $RuO_2 \cdot H_2O$ und 0,5 Mol »Hydroxysäure« (559,3 g einer 11,8%igen Lösung) wurde Chlorgas (etwa 0,5 g/Minute) zugeführt. Die Temperatur wurde auf 0—5°C gehalten, und nachdem etwa 0,7 Mol $Cl_2$ zugegeben waren, wurde die Lösung auf Raumtemperatur erwärmt und filtriert, und es wurden 839 g einer Lösung mit einem Gehalt von 7,05% »Ketosäure« (Ausbeute 91%) und 0,38% »Hydroxysäure« (Ausbeute 4,8%) erhalten.

Beispiel 8

Beispiel 1 wurde, jedoch unter Verwendung von Rutheniumtrichlorid-Hydrat, wiederholt. 350,5 g 11,4%iges NaOCl wurden zu einer Lösung von 511,6 g 12,9%iger »Hydroxysäure« mit einem Gehalt von 0,3 g $RuCl_3 \cdot H_2O$ zugetropft. Man erhielt 818,7 g einer Lösung mit einem Gehalt von 7,25% »Ketosäure« (Ausbeute 91,3%).

Beispiel 9

Es wurde wie in Beispiel 1 beschrieben gearbeitet, jedoch wurden in den Kolben 279,7 g 11,8%ige »Hydroxysäure« eingebracht und der pH-Wert wurde auf 12 eingestellt. Dann wurden 0,3 g $RuO_2 \cdot H_2O$ zugegeben und 76,8 g einer 26%igen NaOCl-Lösung zugetropft. Es wurden 341 g einer Lösung mit einem Gehalt von 9,53% »Ketosäure« (Ausbeute 100%) erhalten.

**Patentansprüche**

1. Verfahren zur Herstellung von 3,3-Dimethyl-2-oxobuttersäure und ihren Salzen durch Oxidation von 3,3-Dimethyl-2-hydroxybuttersäure, dadurch gekennzeichnet, daß man die Umsetzung in wäßrig-alkalischer Lösung mit der zumindest stöchiometrischen Menge eines Salzes der unterchlorigen Säure als Oxidationsmittel in Gegenwart eines Rutheniumkatalysators durchführt und die 3,3-Dimethyl-2-oxobuttersäure gegebenenfalls aus der Lösung ihrer Salze freisetzt.

2. Verfahren nach Anspruch 1, dadurch gekennzeichnet, daß man die Umsetzung in natron-alkalischer Lösung durchführt.

3. Verfahren nach Anspruch 1, dadurch gekennzeichnet, daß man die Umsetzung bei Temperaturen zwischen 40 und 60°C durchführt.

4. Verfahren nach Anspruch 1, dadurch gekennzeichnet, daß man den pH-Wert des Reaktionsmediums auf etwa 9—13, vorzugsweise auf etwa 10—12 einstellt.

5. Verfahren nach Anspruch 1, dadurch gekennzeichnet, daß man das Salz der unterchlorigen Säure in einem Überschuß von 5—15% einsetzt.

6. Verfahren nach Anspruch 1, dadurch gekennzeichnet, daß man den Rutheniumkatalysator in Mengen von etwa 0,01—1 g, vorzugsweise von etwa 0,1—0,5 g pro Mol 3,3-Dimethyl-2-hydroxybuttersäure einsetzt.

7. Verfahren nach Anspruch 1, dadurch gekennzeichnet, daß man als Rutheniumkatalysator Rutheniumdioxidhydrat oder Rutheniumtrichlorid-hydrat einsetzt.

8. Verfahren nach Anspruch 1, dadurch gekennzeichnet, daß man ein Gemisch aus 3,3-Dimethyl-2-hydroxybuttersäure und Rutheniumkatalysator in wäßrig-alkalischer Lösung vorlegt und das Salz der unterchlorigen Säure zufügt.

9. Verfahren nach Anspruch 1, dadurch gekennzeichnet, daß man das Salz der unterchlorigen Säure in situ erzeugt durch Einleiten von Chlorgas in das wäßrig-alkalische, ein Salz der 3,3-Dimethyl-2-hydroxybuttersäure und den Rutheniumkatalysator enthaltende Reaktionsmedium.

## Claims

1. Process for the preparation of 3,3-dimethyl-2-oxobutyric acid and its salts by the oxidation of 3,3-dimethyl-2-hydroxybutyric acid, characterised in that the reaction is carried out in an aqueous alkaline solution with an at least stoichiometric amount of a salt of the hypochlorous acid as oxidizing agent in the presence of a ruthenium catalyst and the 3,3-dimethyl-2-oxobutyric acid is optionally liberated from the solution of its salts.

2. Process according to Claim 1, characterised in that the reaction is carried out in a sodium hydroxide alkaline solution.

3. Process according to Claim 1, characterised in that the reaction is carried out at temperatures between 40 and 60° C.

4. Process according to Claim 1, characterised in that the pH value of the reaction medium is adjusted to about 9—13, preferably about 10—12.

5. Process according to Claim 1, characterised in that an excess of 5—15% of the salt of the hypochlorous acid is used.

6. Process according to Claim 1, characterised in that the ruthenium catalyst is used in amounts of about 0,01—1 g, preferably of about 0,1—0,5 g per mol of 3,3-dimethyl-2-hydroxybutyric acid.

7. Process according to Claim 1, characterised in that ruthenium dioxide hydrate or ruthenium trichloride hydrate are used as the ruthenium catalyst.

8. Process according to Claim 1, characterised in that a mixture of 3,3-dimethyl-2-hydroxybutyric acid and ruthenium catalyst in an aqueous alkaline solution are initially introduced and the salt of the hypochlorous acid is added.

9. Process according to Claim 1, characterised in that the salt of the hypochlorous acid is formed in situ by passing chlorine gas into the aqueous alkaline reaction medium containing a salt of 3,3-dimethyl-2-hydroxy-butyric acid and the ruthenium catalyst.

## Revendications

1. Procédé de fabrication d'acide 3,3-diméthyl-2-oxobutyrique et de ses sels par oxydation de l'acide3,3-diméthyl-2-hydroxybutyrique,caractérisé en ce qu'on exécute la réaction en solution alcaline aqueuse avec la quantité au moins stoechiométrique d'un sel d'acide hypochloreux comme oxydant en présence d'un catalyseur au ruthénium et en ce qu'on met éventuellement en liberté l'acide 3,3-diméthyl-2-oxobutyrique à partir de la solution de ses sels.

2. Procédé selon la revendication 1, caractérisé en ce qu'on exécute la réaction en solution de soude caustique.

3. Procédé selon la revendication 1, caractérisé en ce qu'on exécute la réaction à des températures entre 40 et 60° C.

4. Procédé selon la revendication 1, caractérisé en ce qu'on règle la valeur du pH du milieu de réaction à environ 9—13, de préférence à environ 10—12.

5. Procédé selon la revendication 1, caractérisé en ce qu'on utilise le sel d'acide hypochloreux en un excès de 5—15%.

6. Procédé selon la revendication 1, caractérisé en ce qu'on utilise le catalyseur au ruthénium en des quantités d'environ 0,01—1 g, de préférence d'environ 0,1—0,5 g par mole d'acide 3,3-diméthyl-2-hydroxybutyrique.

7. Procédé selon la revendication 1, caractérisé en ce qu'on utilise comme catalyseur au ruthénium du dioxyde de ruthénium hydraté ou du trichlorure de ruthénium hydraté.

8. Procédé selon la revendication 1, caractérisé en ce qu'on introduit d'avance un mélange d'acide 3,3-diméthyl-2-hydroxybutyrique et de catalyseur au ruthénium en solution alcaline aqueuse et en ce qu'on ajoute le sel d'acide hypochloreux.

9. Procédé selon la revendication 1, caractérisé en ce qu'on engendre le sel d'acide hypochloreux in situ en faisant passer du chlore gazeux dans le milieu de réaction alcalin aqueux contenant un sel d'acide 3,3-diméthyl-2-hydroxybutyrique et le catalyseur au ruthénium.